# FASCICULE DE BREVET EUROPEEN

(11) **EP 0 310 483 B1**
(45) Date de publication et mention de la délivrance du brevet: **24.02.1993**
(21) Numéro de dépôt: 88402415.9
(22) Date de dépôt: 26.09.1988
(51) Int. Cl.: A61F 2/42

(54) **Prothèse d'articulation métacarpo-phalangienne ou inter-phalangienne de doigts**
Prosthetisches Metacarpophalangeal- oder Interphalangeal-Fingergelenk
Metacarpal-phalangeal or inter-phalangeal articulation prosthesis of the finger

(30) Priorité: 28.09.1987 FR 8713370
(43) Date de publication de la demande: 05.04.1989
(73) Titulaire: Saffar, Philippe, Dr., F-92299 Neuilly-sur-Seine (FR)
(72) Inventeur: Saffar, Philippe, Dr., F-92299 Neuilly-sur-Seine (FR)
(74) Mandataire: Colas, Jean-Pierre

(56) Documents cités:
- FR-A- 2 417 292
- FR-A- 2 465 470
- GB-A- 1 530 301
- US-A- 4 352 212

## Description

L'invention concerne une prothèse d'articulation de doigt et plus particulièrement une prothèse métacarpo-phalangienne ou inter-phalangienne, comportant deux éléments coopérants, constituant l'articulation proprement dite, chacun des éléments étant muni d'une queue implantable dans les canaux médullaires des os, l'un des éléments de l'articulation se présentant sous la forme d'un boîtier ouvert, selon le plan sagittal, sur un côté palmaire et un de ses côtés adjacents, l'autre desdits côtés adjacents portant la queue d'implantation, l'autre élément d'articulation comportant une tête susceptible de se loger dans le boîtier et tournant contre une partie concave prévue dans le fond du boîtier, la tête étant séparée de la queue d'implantation par une collerette sensiblement perpendiculaire à l'axe de la queue.

La destruction des articulations des doigts, causée par un traumatisme, par des rhumatismes dégénératifs ou inflammatoires, et plus rarement par une infection, provoque un handicap sérieux que l'on a tenté de combler par l'implantation de prothèses métacarpo-phalangiennes ou inter-phalangiennes.

C'est ainsi que le brevet français 2.242.067 décrit une prothèse constituée d'une pièce en matière flexible présentant transversalement, en son milieu, une partie amincie formant une charnière flexible. Les extrémités de la pièce constituent des queues d'implantation qui sont enfoncées dans les canaux médullaires.

De telles prothèses permettent de retrouver une certaine mobilité mais présentent toutefois une limitation de la mobilité de l'articulation. On a en outre observé de nombreux cas de réactions osseuses autour des queues des prothèses, par suite de l'usure du matériau et de l'émission de corps étranger dans l'os. L'inconvénient le moins grave qui résultait du glissement répété de la queue dans le canal médullaire était l'agrandissement éventuel de ce dernier.

Le brevet français 2.450.600 évitait cet inconvénient en prévoyant sur les éléments d'articulation des broches susceptibles de coulisser librement dans des douilles formées dans des tampons scellés dans les canaux médullaires. Le maintien en place de l'articulation est alors assuré uniquement par les ligaments et tendons.

L'articulation est constituée d'une tête de forme cylindrique, montée sur la broche coulissant dans un des embouts médullaires, coopérant avec la surface concave d'une coupelle plastique, logée dans un boîtier monté sur la broche coulissant dans l'autre embout. L'axe articulaire est l'axe longitudinal de la tête cylindrique. Afin d'obtenir un certain degré de liberté dans le plan radio-cubital de l'articulation, les extrémités de la tête cylindrique sont hémisphériques. Le maintien et le rappel de l'articulation se fait par les ligaments et tendons naturels.

Comme les prothèses à charnière, celle-ci présente des pertes d'extension et de flexion dues au fait que les tendons extenseurs et fléchisseurs ne peuvent assurer leur fonction d'adaptation à la longueur variable des doigts entre les positions d'extension et de flexion.

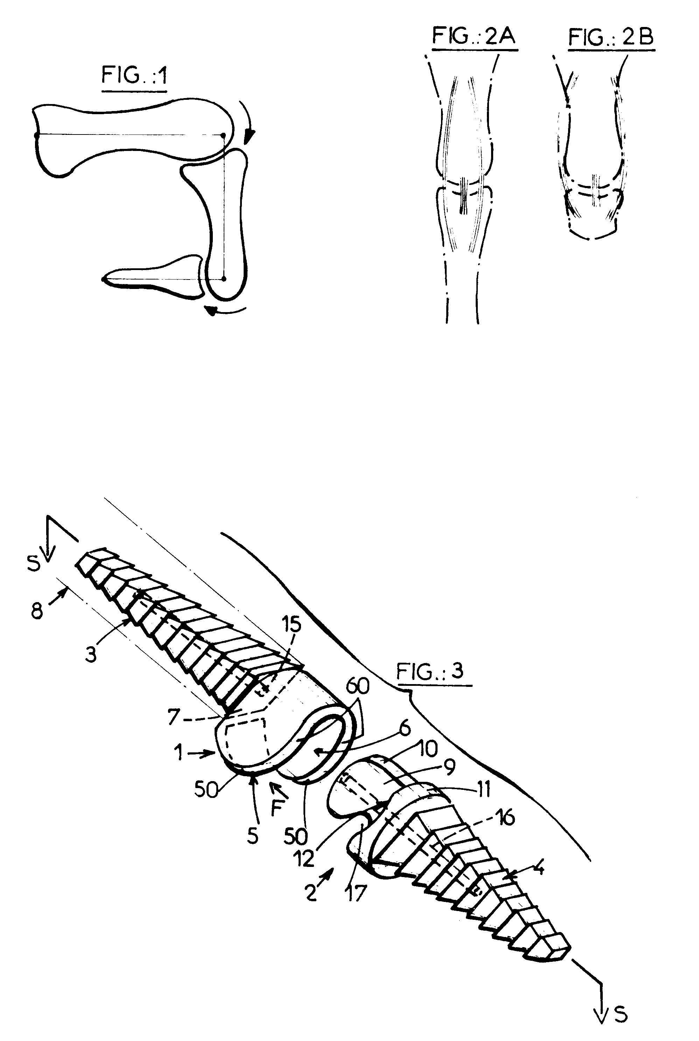

En effet, si l'on mesure la face dorsale d'un doigt, on constate que le doigt est plus court en extension qu'en flexion. Cette différence s'explique par le fait que les phalanges en flexion se mettent les unes devant les autres (figure 1) et ne tournent pas autour d'un axe unique. A chaque angle de flexion correspond un axe de rotation. La partie distale des métacarpiens ainsi que des phalanges, présente dans le plan sagittal, une tête asymétrique par rapport à l'axe du métacarpien ou de la phalange faisant saillie dans la direction palmaire.

Les mécanismes de compensation existant sur les tendons fléchisseurs et les tendons extenseurs permettent le rattrapage des différences de longueur par exemple, par le rapprochement et l'écartement des bandelettes latérales des extenseurs en extension et en flexion (figures 2A, 2B).

On connaît aussi du document FR-A-2.417.292 une prothèse d'articulation métacarpienne ou du doigt qui comprend un premier élément d'articulation ancré par une queue dans un premier os, avec une entretoise médiane de part et d'autre de laquelle sont disposées des surfaces de glissement cintrées, ainsi que d'un deuxième élément d'articulation pareillement ancré par une queue dans un second os et muni de deux patins qui surplombent l'entretoise, prennent appui sur les surfaces de glissement et dont les faces sont dirigées vers le premier os. L'entretoise du premier élément d'articulation est traversée de part en part par un axe transversal fixe axialement, qui est déporté excentriquement par rapport au bombement des surfaces de glissement. Les extrémités de l'axe transversal s'engagent avec jeu dans des rainures de guidage des patins, rainures qui sont sensiblement parallèles à la surface des patins, font vis-à-vis à l'entretoise et sont ouvertes à leur extrémité tournée vers l'intérieur de l'articulation, tandis que, par leur extrémité opposée, elles forment une butée pour l'axe transversal en position déployée de l'articulation.

La présence de rainures dans les patins a pour effet d'accroître l'épaisseur transversal de ceux-ci, et donc l'épaisseur correspondante de la prothèse ce qui est défavorable compte tenu des contraintes d'encombrement qui pèsent sur les prothèses de doigt. En outre un basculement de l'axe par rapport aux rainures dans lesquelles il s'engage peut provoquer un blocage de l'articulation. Enfin la réalisation d'un tel axe et de telles rainures, dans des pièces de petites dimensions accroît les coûts de fabrication.

L'invention vise à l'obtention d'une articulation multiaxiale correspondant à la physiologie des articulations normales et qui ne présente pas les inconvénients mentionnés ci-dessus.

La prothèse selon l'invention comprend deux éléments coopérants, constituant l'articulation proprement dite, chacun des éléments étant muni d'une queue implantable dans les canaux médullaires des os, l'un des éléments de l'articulation se présentant sous la forme d'une chape ouverte selon le plan sagittal, sur un côté palmaire et un de ses côtés adjacents, l'autre desdits côtés adjacents portant la queue d'implantation, l'autre élément d'articulation comportant une tête susceptible de se loger dans la chape et de tourner, au moins partiellement, contre une partie concave prévue dans le fond de cette chape pour rester en contact avec ce fond dans la position d'extension de la prothèse, le plan de symétrie de la tête correspondant au plan sagittal et celle-ci étant séparée d'une queue d'implantation par une collerette perpendiculaire à l'axe de la queue et présentant, parallèlement au plan sagittal, deux saignées concaves conçues pour glisser contre deux bords correspondants de la chape sous l'action de forces faisant tourner un élément d'articulation par rapport à l'autre en chargeant ces saignées contre ces bords, la courbure des saignées concaves correspondant sensiblement à la courbure des bords faisant face à la collerette dans la position d'extension de la prothèse, prothèse caractérisée en ce que la chape comporte un côté dorsal semi-cylindrique et en ce que la tête présente deux faces latérales sensiblement planes et parallèles au plan sagittal séparées par une distance légèrement inférieure à celles de deux faces internes sensiblement planes de la chape et disposées chacune en regard d'une face latérale de la tête quand celle-ci est logée dans la chape, de manière à guider alors le déplacement relatif des éléments d'articulation dans un plan sensiblement sagittal.

Les explications et figures données ci-après à titre d'exemples permettront de comprendre comment l'invention peut être réalisée.

La figure 1 est une vue dans le plan sagittal des articulations inter-phalangiennes en flexion.

Les figures 2A et 2B montrent la position des bandelettes du tendon extenseur dans le mécanisme de compensation, les phalanges étant respectivement en extension et en flexion.

La figure 3 est une vue en perspective et éclatée d'une prothèse selon l'invention.

La figure 4 est une vue en coupe partielle de la prothèse de la figure 3 en extension (en trait plein) et en flexion (en trait interrompu).

La figure 5 est une vue selon F de la figure 3.

La figure 6 est une vue selon F d'une autre forme de réalisation de la figure 3.

La figure 7 est une vue en perspective éclatée d'une autre forme de réalisation de la prothèse selon l'invention.

La figure 3 est une vue en perspective et éclatée d'une première forme de réalisation d'une articulation selon l'invention.

Cette prothèse est plus particulièrement destinée au remplacement des articulations métacarpo-phalangiennes et inter-phallangiennes après résection des parties osseuses articulaires distale et proximale et préparation des diaphyses.

L'articulation est constituée de deux éléments 1,2 munis de queues de scellement 3,4 destinées à être implantées dans les canaux médullaires des os.

L'élément d'articulation 1 se présente sous la forme d'un boîtier formant chape dont le plan de symétrie principal est le plan sagittal S. Le boîtier est ouvert selon ce même plan sur le côté palmaire 5 et un des côtés adjacents 6 au côté palmaire. L'autre côté adjacent 7 au côté palmaire porte perpendiculairement à sa face, la queue de scellement 3.

Selon la forme de réalisation représentée figures 3, 5 et 6, le côté dorsal du boîtier-chape est de forme approximativement cylindrique d'axe sensiblement parallèle à l'axe de la queue et présente en coupe transversale une courbure correspondant approximativement à celle des diaphyses 8 qu'il prolonge.

Les bords 50 des ailes de la chape, parallèles au plan sagittal du côté palmaire 5, sont asymétriques du côté dorsal de la chape par rapport à l'axe de la queue d'implantation correspondant sensiblement à l'axe des diaphyses métacarpiens ou phalangiennes et forment dans le plan sagittal une courbe convexe raccordée selon une courbe continue avec les bords également convexes 60 du côté 6 de la chape. La courbe convexe formée par les côtés 5 et 6 ne présente pas de centre de courbure unique.

La courbure des bords est prévue de manière que le déplacement longitudinal de la phalange de la position en extension à la position en flexion soit comparable au déplacement qui se produit dans l'articulation physiologique. Ceci se traduit pour la chape par une hauteur supérieure à sa longueur, la longueur étant déterminée selon l'axe de la queue d'implantation et la hauteur, perpendiculairement à la longueur, à la partie la plus haute au sommet de la convexité des bords 50.

Dans l'exemple de réalisation figurée, le boîtier chape, fixé de préférence sur le métacarpien pour les prothèses métacarpo-phalangiennes et sur la première phalange pour les articulations inter-phalangiennes proximales, sera désignée sous le terme de "pièce proximale", tandis que l'élément d'articulation 2 sera désigné par le terme de "pièce distale".

L'élément d'articulation 2 ou pièce distale, comporte une tête 9 dont le plan de symétrie correspond au plan sagittal. Les dimensions de la tête sont telles que sa largeur correspond approximativement à la largeur intérieure la plus faible, comprise entre les côtés parallèles de la chape 1 et que sa partie dorsale 10 est en contact avec le fond de la chape dans la position d'extension.

La tête est séparée de la queue d'implantation 4 par une collerette 11 perpendiculaire à l'axe de la queue et qui présente de part et d'autre de la tête, parallèlement au plan sagittal, des saignées concaves 12 dont la courbure correspond sensiblement à la courbure de la partie des bords 60 de la pièce proximale 1, faisant face à la collerette 11, dans la position d'extension. Ces saignées, ainsi que la forme de la tête, empêchent la rotation et les mouvement latéraux.

Selon la forme de réalisation représentée, l'extrémité de la tête a un profil de came convexe prévu pour coopérer au moins partiellement avec un chemin de glissement 13, formé à l'intérieur de la chape, sur le côté dorsal et la partie 7 portant la queue d'implantation 3. Pendant le glissement de la pièce distale le long du bord de la chape de la pièce proximale, le contact est maintenu au moins partiellement, d'une part, entre la tête à profil de came 9 et le chemin de glissement 13 et d'autre part, entre les saignées concaves 12 de la collerette 11 et les bords convexes 50 et 60 de la chape. Les côtés sagittaux de la chape empêchent les mouvements de latéralité et remplacent les ligaments latéraux (figure 5).

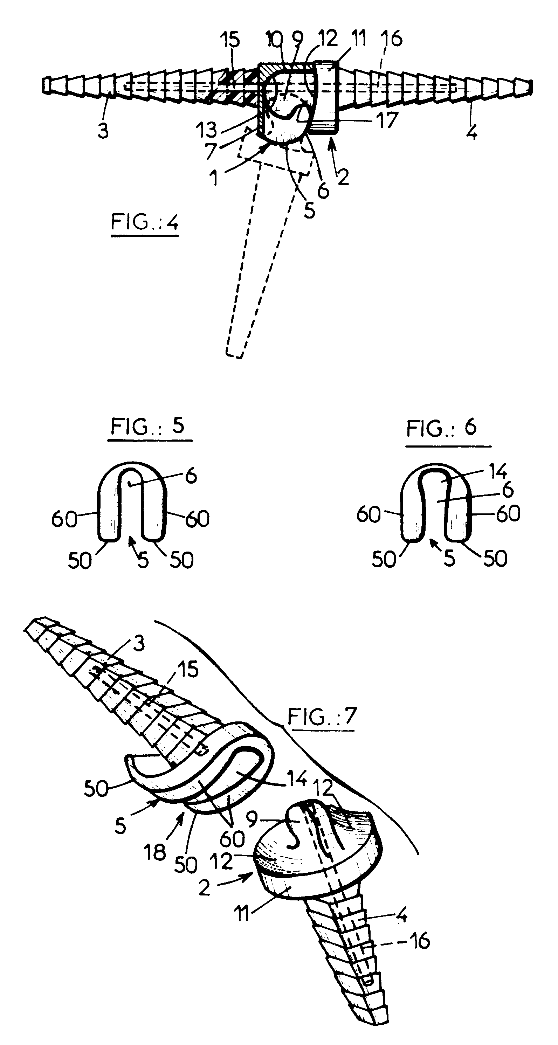

Selon une forme de réalisation plus particulièrement adaptée aux articulations métacarpo-phalangiennes qui présentent des mouvements de latéralité en extension, on forme un évasement 14 (figure 6) à la partie supérieure voisine du fond de la chape, afin de permettre à la pièce distale, des mouvements de latéralité modérés correspondant aux mouvements physiologiques. Cet évasement peut être combiné en donnant une forme complémentaire, présentant un jeu, à la tête 9 pour éviter un déboîtement des pièces proximale 1 et distale 2.

Selon une forme de réalisation représentée figures 3, 4, les queues d'implantation 3 et 4 sont pyramidales à base rectangulaire. La section rectangulaire des queues est prévue de manière à pouvoir pénétrer dans les canaux médullaires de forme ovale des métacarpiens et des phalanges. Les espaces existant entre les faces des queues et le canal médullaire, permettent la reconstitution osseuse assurant le maintien de la prothèse.

Les queues sont prévues, selon un exemple de réalisation, en polyéthylène renforcé axialement sur au moins une partie de leur longueur par une tige métallique 15, 16.

La chape est réalisée en un métal physiologiquement compatible, tel par exemple, le titane et porte éventuellement sur sa partie dorsale un revêtement en matériau céramique.

La tige de renfort 15 est alors également en titane et est fixée dans la paroi métallique 7 de la chape.

La pièce distale, comportant la tête, la collerette et la queue, est réalisée en polyéthylène renforcé axialement sur au moins une partie de leur longueur par une tige métallique. De préférence, la tige affleure l'extrémité de la tête, où elle forme une surface de glissement du profil de came sur le chemin de glissement de la chape.

La limitation de la longueur de la tige à environ la moitié de longueur des queues, permet l'ajustement de la longueur des queues à la profondeur de la partie préparée du canal médullaire par simple sectionnement de la partie de polyéthylène superflu.

La tige de renfort a pour but d'éviter, lors de la mise en place ou du retrait des parties proximale et distale, la séparation par rupture de la chape ou de la tête d'avec la queue d'implantation. Eventuellement la tige de renfort et la queue sont solidarisées par une goupille les traversant afin d'empêcher toute rotation entre lesdites deux pièces.

La tête 9 présente sur son bord palmaire, une encoche 17 qui permet d'augmenter l'angle de flexion (figure 4) de la pièce distale avant qu'elle ne parvienne en butée avec le côté 7 de la chape.

Selon un deuxième exemple de réalisation représenté figure 7, la chape est remplacée par un bouclier 18 dont la surface courbe correspond aux bords de forme convexes 50 et 60 de la chape de l'exemple précédent. Cette forme de réalisation est utilisée lorsque la plaque palmaire de l'articulation est en bon état et peut être conservée. Dans cette nouvelle forme de réalisation, les éléments semblables à ceux du premier exemple, portent les mêmes références.

La technique de mise en place des prothèses métacarpo-phalangienne est connue et se pratique soit par la voie d'abord dorsale soit par la voie d'abord antérieure.

La voie d'abord antérieure est recommandée dans la polyarthrite rhumatoïde ou si l'on soupçonne une atteinte de la plaque palmaire importante avec subluxation antérieure de l'articulation.

La mise en place des prothèses inter-phalangiennes se fera par voie latérale, à l'union des peaux dorsopalmaires du côté interne pour le deuxième et troisième doigts et du côté externe pour le quatrième et cinquième.

## Revendications

1. Prothèse d'articulation de doigt et plus particulièrement une prothèse métacapo-phalangienne ou inter-phalangienne comportant deux éléments (1,2) coopérants, constituant l'articulation proprement dite, chacun des éléments (1,2) étant muni d'une queue implantable (3,4) dans les canaux médullaires des os, l'un (1) des éléments de l'articulation se présentant sous la forme d'une chape ouverte selon le plan sagittal (S), sur un côté palmaire et un de ses côtés adjacents, l'autre desdits côtés adjacents portant la queue d'implantation (3), l'autre élément d'articulation (2) comportant une tête (9) susceptible de se loger dans la chape et tourner au moins partiellement contre une partie concave prévue dans le fond de cette chape pour rester en contact avec ce fond dans la position d'extension de la prothèse, le plan de symétrie de la tête correspondant au plan sagittal et celle-ci étant séparée d'une queue d'implantation (4) par une collerette (11) perpendiculaire à l'axe de la queue et présentant, parallèlement au plan sagittal, deux saignées concaves (12) conçues pour glisser contre deux bords (50) correspondants de la chape sous l'action de forces faisant tourner un élément d'articulation par rapport à l'autre en chargeant ces saignées contre ces bords, la courbure des saignées concaves correspondant sensiblement à la courbure des bords faisant face à la collerette dans la position d'extension de la prothèse, caractérisée en ce que la chape comporte un côté dorsal semi-cylindrique et en ce que la tête (9) présente deux faces latérales sensiblement planes et parallèles au plan sagittal (S) séparées par une distance légèrement inférieure à celle de deux faces internes sensiblement planes de la chape et disposées chacune en regard d'une face latérale de la tête quand celle-ci est logée dans la chape, de manière à guider alors le déplacement relatif des éléments d'articulation dans un plan sensiblement sagittal.

2. Prothèse selon la revendication 1, caractérisée en ce que la chape a une forme de bouclier (18) présentant une surface courbe multicentrique, susceptible de coopérer avec les saignées concaves (12) lors de l'extension et de la flexion des doigts.

3. Prothèse selon la revendication 1 ou 2, caractérisé en ce que la tête (9) présente un profil de came convexe prévu pour coopérer au moins partiellement avec un chemin de glissement (13), formé à l'intérieur de la chape, sur le côté dorsal et la partie (7) portant la queue d'implantation (3), lors de la flexion des doigts.

4. Prothèse selon l'une des revendications 1 à 3, caractérisée en ce que les queues d'implantation (3, 4) sont pyramidales à base rectangulaire.

5. Prothèse selon l'une des revendications 1 à 4, caractérisée en ce que les queues sont en polyéthylène.

6. Prothèse selon la revendication 5, caractérisée en ce que la queue comporte, selon son axe longitudinal et sur au moins une partie de sa longueur une tige métallique de renfort (15).

7. Prothèse selon la revendication 6, caractérisée en ce que la tige de renfort est fixée à une extrémité, à un côté (7) de la chape (1).

8. Prothèse selon la revendication 6, caractérisée en ce que la tige de renfort traverse la tête (9).

9. Prothèse selon l'une des revendications précédentes, caractérisée en ce que l'on forme un évasement (14) à la partie supérieure du fond de la chape (1) ou du bouclier permettant les mouvements latéraux en extension du doigt.

10. Prothèse selon l'une des revendications précédentes, caractérisé en ce que la partie dorsale de la chape comporte un revêtement en matériau céramique.

11. Prothèse selon la revendication 6, caractérisé en ce que les tiges de renfort (15) et les queues d'implantation (3) en polyéthylène sont solidarisées par une goupille anti-rotation.

12. Prothèse selon la revendication 9, caractérisée en ce que la tête (9) a une forme complémentaire de la surface adjacente de l'évasement (14) de la chape (1), ladite forme ayant du jeu par rapport à l'évasement.

## Claims

1. A prosthesis for finger joint and, more particularly, a metacarpophalangeal or interphalangeal prosthesis comprising two cooperating components (1,2) constituting the actual joint, each of the components (1,2) being equipped with a stem which can be implanted in the medullary canals of the bones, one (1) of the joint components being in the form of a socket opened, in the sagittal plane (S), on a palmar side and one of its adjacent sides, the other of the said adjacent sides bearing the implant stem (3), the other joint component (2) comprising a head (9) capable of being accomodated in the socket and turning against a concave part provided in the bottom of the socket to remain in contact with said bottom in the extension position of the prosthesis, the plane of symmetry of the head corresponding to the sagittal plane and the head being separated from an implant stem (4) by a collar (11) perpendicular to the axis of the stem and showing, parallely to the sagittal plane, two concave cut-ins (12) designed to slide against two corresponding edges (50) of the socket under forces rotating one joint component with respect to the other while loading said cut-ins against said edges, the cut-ins curvature substantially corresponding to the one of said edges facing the collar in the extension position of the prosthesis, characterized in that the socket comprises a semicylindrical dorsal side and in that the head (9) comprises two substantially flat lateral sides parallel to the sagittal plane (S), spaced apart a distance slightly less than that of the substantially flat internal sides of the socket and each facing a lateral side of the head when the latter is inserted into the socket, so as to guide the relative movement of the joint components in a substantially sagittal plane.

2. The prosthesis as claimed in claim 1, characterized in that the socket is shaped as a shield (18) having a multicentric curved surface capable of cooperating with the concave cut-ins (12) during extension and flexion of the fingers.

3. The prosthesis as claimed in claim 1 or 2, characterized in that the head (9) has a convex cam profile provided for cooperating at least partially with a slide track, formed inside the socket, on the dorsal side and the part bearing the implant stem, during flexion of the fingers.

4. The prosthesis as claimed in claims 1 to 3, characterized in that the implant stems (3,4) are pyramidal with a rectangular base.

5. The prosthesis as claimed in claims 1 to 4, characterized in that the tails are of polyethylene.

6. The prosthesis as claimed in claim 5, characterized in that the tail has, in its longitudinal axis and over at least a part of its length, a metallic strengthening rod (15).

7. The prosthesis as claimed in claim 6, characterized in that the strengthening rod is fixed at one end to a side (7) of the socket (1).

8. The prosthesis as claimed in claim 6, characterized in that the strengthening rod passes through the head (9).

9. The prosthesis as claimed in anyone of the proceeding claim, characterized in that a widening (14) is formed in the upper part of the bottom of the socket (1) or of the shield permitting lateral movements during extension of the finger.

10. The prosthesis as claimed in anyone of the proceeding claims, characterized in that the dorsal part of the socket comprises a clothing of ceramic material.

11. The prosthesis as claimed in claim 6, characterized in that the strengthening rods (15) and the implant tails (3) of polyethylene are made integral by an antirotation pin.

12. The prosthesis as claimed in claim 9, characterized in that the head (9) is complementarily shaped with respect to the adjacent surfaces of the widening (14) of the socket (1), said shape being loosely adjusted with respect to said widening.

## Patentansprüche

1. Fingergelenkprothese, insbesondere Metacarpophalangial- oder Interphalangialprothese, welche zwei zusammenwirkende Elemente (1, 2) aufweist, die das eigentliche Gelenk bilden, wobei jedes der Elemente (1, 2) mit einem in den Knochenmarkkanal implantierbaren Schaft (3, 4) versehen ist, wobei eines (1) der Gelenkelemente in der Form eines Gehäuses ausgebildet ist, das in der Pfeilebene (S) offen ist, und zwar auf der Palmarseite (Handinnenseite) und auf einer der benachbarten Seiten, wobei die andere der benachbarten Seiten den Implantationsschaft (3) trägt, wobei das andere Gelenkelement (2) einen Kopf (9) aufweist, der in dem Gehäuse aufgenommen werden kann und sich zumindest teilweise gegen einen konkaven Teil verdrehen kann, welcher im hinteren oder Bodenteil des Gehäuses vorgesehen ist, so daß er mit dem Bodenteil in der Position der Streckung der Prothese in Kontakt bleibt, wobei die Symmetrieebene des Kopfes der Pfeilebene entspricht und wobei der Kopf von dem Implantationsschaft (4) durch einen Flansch (11) senkrecht zu der Schaftachse getrennt ist, welcher parallel zu der Pfeilebene zwei konkave Aussparungen oder Senken (12) aufweist zum Gleiten auf zwei entsprechenden Rändern (50) des Gehäuses, und zwar durch die Wirkung von Kräften die ein Gelenkelement zur Drehung relativ zu dem anderen veranlassen unter Belastung der Aussparungen gegen die Ränder, wobei die Krümmung der konkaven Aussparungen im wesentlichen der Krümmung der Ränder entspricht, die gegenüber dem Flansch in der Position der Streckung der Prothese liegen, dadurch gekennzeichnet, daß das Gehäuse eine halbzylindrische Dorsalseite (Rückenseite) aufweist, und daß der Kopf (9) zwei im wesentlichen ebene Seitenflächen parallel zu der Pfeilebenen (S) aufweist, die durch einen Abstand voneinander getrennt sind, der etwas geringer ist als derjenige zwischen den im wesentlichen ebenen Innenseiten des Gehäuses, die jeweils gegenüberliegend zu einer der Seitenflächen des Kopfes angeordnet sind, wenn dieser in dem Gehäuse aufgenommen ist, um so die relative Versetzung der Gelenkelemente im wesentlichen in einer Pfeilebene zu führen.

2. Prothese nach Anspruch 1, dadurch gekennzeichnet, daß das Gehäuse eine Schildform (18) aufweist, die eine multizentrisch gekrümmte Oberfläche besitzt und mit den konkaven Aussparungen (12) bei Streckung und Beugung der Finger zusammenwirken kann.

3. Prothese nach Anspruch 1 oder 2, dadurch gekennzeichnet, daß der Kopf (9) ein konvexes Nockenprofil aufweist zum Zusammenwirken zumindest teilweise mit einer Gleitbahn (13), die im Innern des Gehäuses gebildet ist, und zwar auf der Rükkenseite und dem Teil (7), der den Implantationsschaft (3) trägt, bei Beugung der Finger.

4. Prothese nach einem der Ansprüche 1 bis 3, dadurch gekennzeichnet, daß die Implantationsschäfte (3, 4) pyramidenförmig mit einer rechteckigen Basis sind.

5. Prothese nach einem der Ansprüche 1 bis 4, dadurch gekennzeichent, daß die Schäfte aus Polyethylen bestehen.

6. Prothese nach Anspruch 5, dadurch gekennzeichnet, daß der Schaft entlang seiner Längsachse und zumindest auf einem Teil seiner Länge eine Verstärkunsstange (15) aus Metall aufweist.

7. Prothese nach Anspruch 6, dadurch gekennzeichnet, daß die Verstärkunsstange mit einem Ende an einer Seite (7) des Gehäuses (1) befestigt ist.

8. Prothese nach Anspruch 6, dadurch gekennzeichnet, daß die Verstärkungsstange den Kopf (9) durchdringt.

9. Prothese nach einem der vorhergehenden Ansprüche, dadurch gekennzeichnet, daß eine Ausbauchung (14) im oberen Teil des Bodenteils des Gehäuses (1) oder im Schild vorgesehen ist, die seitliche Bewegungen bei Streckung des Fingers gestattet.

10. Prothese nach einem der vorhergehenden Ansprüche, dadurch gekennzeichnet, daß der Rückenteil des Gehäuses eine Verkleidung oder Ummantelung aus keramischen Material aufweist.

11. Prothese nach Anspruch 6, dadurch gekennzeichnet, daß die Verstärkungsstangen (15) und die Implantationsschäfte (3) aus Polyethylen zum Schutz gegen Verdrehen mittels eines Splints miteinander verbunden sind.

12. Prothese nach Anspruch 9, dadurch gekennzeichnet, daß der Kopf (9) eine Form hat, die komplementär zu der benachbarten Oberfläche der Ausbauchung (14) des Gehäuses (1) ist, wobei die Form bezüglich der Ausbauchung Spiel hat.
